# EUROPÄISCHE PATENTSCHRIFT

(11) **EP 3 431 123 B1**
(45) Veröffentlichungstag und Bekanntmachung des Hinweises auf die Patenterteilung: **23.09.2020**
(21) Anmeldenummer: 17181771.1
(22) Anmeldetag: 18.07.2017
(51) Int. Cl.: A61M 5/34, A61M 5/28, A61M 5/31

(54) **VERFAHREN ZUR HERSTELLUNG EINER SPRITZE MIT EINEM STECHMITTEL**
METHOD FOR PRODUCING A SYRINGE WITH A PUNCTURING MEANS
PROCÉDÉ DE FABRICATION D'UNE SERINGUE COMPRENANT UN MOYEN DE PERÇAGE

(43) Veröffentlichungstag der Anmeldung: 23.01.2019
(73) Patentinhaber: Gerresheimer Regensburg GmbH, 93047 Regensburg (DE); Gerresheimer Bünde GmbH, 32257 Bünde (DE)
(72) Erfinder: BAUMANN, Manfred, 92718 Schirmitz (DE); PETERSEN, Claudia, 30167 Hannover (DE); WIGLENDA, Michael, 92699 Irchenrieth (DE)
(74) Vertreter: Hannke, Christian

(56) Entgegenhaltungen:
- EP-A2- 1 364 670
- WO-A1-2017/060052
- DE-A1-102012 101 948
- DE-A1-102015 117 212

## Beschreibung

Die Erfindung betrifft ein Verfahren zur Herstellung einer Spritze mit einem Stechmittel.

Unter einer Spritze wird ein medizinischer Behälter verstanden, welcher an seinem distalen Ende mit einem Stechmittel ausgestattet ist. Es ist auch denkbar, dass das Verfahren auf weitere medizinische Glasbehälter anwendbar ist. Unter einem Stechmittel soll eine Kanüle, eine Nadel oder Ähnliches verstanden werden. Ferner bezieht sich die Erfindung vorwiegend auf Glasspritzen, wobei eine Anwendung im Bereich der Kunststoffspritze durchaus denkbar wäre.

Derartige Spritzen für medizinische Anwendungen umfassen einen hohlzylindrischen Spritzenkörper, in welchem das zu applizierende Medium sich befindet und welcher an einem distalen Ende in einen hohlzylindrischen Endabschnitt ausläuft. Der Innendurchmesser des Endabschnitts ist dabei kleiner als der Innendurchmesser des Spritzenkörpers. Ferner ist der Außendurchmesser des Spritzenkörpers größer als der Außendurchmesser des Endabschnitts. Dieser Endabschnitt ist in der Regel konisch ausgebildet. Ein solcher Endabschnitt wird demnach oft auch als Düse oder Spritzenkonus bezeichnet. Am proximalen Ende des Spritzenkörpers ist in der Regel ein Kolben mit einem Stopfen einführbar mittels welchem das Medium applizierbar ist.

Die Begriffe "distal" und "proximal" sind derart zu verstehen, dass das distale Ende der Spritze näher an dem Applikationsort ist und das proximale Ende weiter weg von dem Applikationsort. Analog sind die Begriffe "distale und proximale Richtung" zu verstehen.

Die Herstellung vorfüllbarer Ganzglasspritzen erfolgt in der Regel durch Erzeugen von Abschnitten von Glasröhren, die als Halbzeug dienen, und nachfolgender Umformung bei hohen Temperaturen. Bei der Umformung werden Formwerkzeuge eingesetzt, die eine ausreichende Wärmeformbeständigkeit und Verschleißfestigkeit bei gleichzeitig hoher Duktilität aufweisen. Die Glasspritzen werden in der Regel aus sehr chemikalien- und temperaturbeständigen Gläsern wie Borosilikatglas oder Quarzglas hergestellt. Der Spritzenkonus weist dabei einen Kanal auf, welcher die Kanüle mit dem Hohlraum der Spritze, in dem das zu verabreichende Medium aufbewahrt wird, verbindet.

Dabei erfolgt die Herstellung des Spritzenkonus durch abschnittsweises Aufheizen des Glasrohlings. Ist das Glas in einem formbaren Zustand, wird die äußere Formgebung mittels eines zweiten Formwerkzeugs und die innere Formgebung durch Gegenhalten eines sogenannten Formstifts oder Formdorns, welcher ein Bestandteil eines ersten Formwerkzeugs ist, hergestellt.

Der Spritzenkonus kann unter anderem als ein sogenannter Luer-Konus ausgebildet werden, auf welchem ein entsprechendes Stechmittel aufsetzbar ist. Vorgefüllt auslieferbare Spritzen werden jedoch oft mit sogenannten "Staked-in Nadeln (SIN)" versehen. Hier wird die Kanüle in dem Kanal üblicherweise durch eine Klebeverbindung befestigt. In der Regel ist es notwendig, dass der Kanal der SIN-Spritzen einen kleineren Durchmesser als bei Spritzen mit einem Luer Konus aufweist, um das Einkleben der Kanüle zu erleichtern.

Die Kanüle wird hierbei zunächst in den distalen Endabschnitt der Spritze eingebracht und anschließend mit einem Kleber in dem Endabschnitt fixiert. Üblicherweise wird ein organischer Klebstoff verwendet, der mit UV-Licht aushärtbar ist. Die mit der Kanüle versehene Spritze wird dann sterilisiert und mit dem entsprechenden Medium befüllt. Das Befüllen kann direkt im Anschluss oder bei einem entsprechenden pharmazeutischen Unternehmen erfolgen. Nach dem Befüllen wird der Stopfen am proximalen Ende des Spritzenkörpers eingesetzt.

Im Stand der Technik besteht über die gesamte Länge des Endabschnitts des Spritzenkörpers die Klebeverbindung zwischen der Kanüle und einer inneren Oberfläche des Endabschnitts. Nach Befüllung mit dem Medium steht dieses also in direktem Kontakt mit dem Klebstoff. Es können somit Bestandteile des Klebstoffes in das Medium/ Medikament eindiffundieren beziehungsweise mit diesem interagieren. Dieser Effekt kann weiterhin noch durch ungenügend ausgehärtete Klebstoffreste verstärkt werden. Moderne Wirkstoffe können durch diese Verunreinigungen stark negativ beeinflusst werden. Derart komplexe und empfindliche Medikamente müssen stärker denn je vor möglichen Kontaminationen bewahrt werden, um deren optimale Wirkungen zu gewährleisten.

In dem Dokument DE10 2010 045 095 B4 wird eine Lösung dieser Problematik präsentiert, in der gänzlich auf einen Klebstoff verzichtet wird. Das Glas wird an der Innenseite der Konusöffnung durch Strahlung erneut aufgeschmolzen und somit eine stoffschlüssige Verbindung zwischen dem Glaskörper und der Kanüle erreicht. Dies hat den Nachteil, dass ein entsprechend modifiziertes Glas, welches elektromagnetische Strahlung entsprechend absorbieren kann, zu verwenden ist.

In dem Dokument EP1370314B1 wird die Problematik durch einen in der Konusöffnung befindlichen Anschlag, an welchem die Kanüle abdichtet, gelöst. Die Konusöffnung wird nach dem Einführen mit Kleber gefüllt, um die erforderlichen Auszugskräfte für die Kanüle gewährleisten zu können. Nachteilig ist hier die präzise und demnach kostenintensive Formung des inneren Anschlages innerhalb der Konusöffnung. Die Präzision in der Fertigung dieses Anschlages ist dabei entscheidend, ob Kleber in das Innere des Spritzenzylinders gelangen kann. Das Dokument EP 1 364 6701 offenbart, dass zunächst die Kanüle in den Nadelkanal eingesetzt wird. Anschließend wird der Endabschnitt im Bereich der Aufweitung erwärmt.

Der Erfindung liegt daher die Aufgabe zugrunde, ein Verfahren zur Herstellung einer Spritze mit einem Stechmittel bereitzustellen, welches eine einfache Fixierung des Stechmittels in der distalen Öffnung des Endabschnitts gewährleistet und gleichzeitig Kontaminationen innerhalb des Spritzenkörpers vermeidet. Ferner ist es Aufgabe der Erfindung eine entsprechende Spritze zur Verfügung zu stellen.

Die Aufgabe wird gelöst durch ein Verfahren zur Herstellung einer Spritze mit einem Stechmittel umfassend folgende Schritte:
a) Bereitstellen eines Spritzenkörpers mit einem distalen Endabschnitt, welcher einen inneren Kanal, der in einer distalen Öffnung mündet, aufweist, wobei der distale Endabschnitt sich in einem formbaren Zustand befindet;
b) Bereitstellen eines Stechmittels;
c) Einführen eines proximalen Abschnitts des Stechmittels durch die distale Öffnung in den inneren Kanal des distalen Endabschnitts;
d) Formen des distalen Endabschnitts mittels eines ersten Formwerkzeugs derart, dass eine innere Oberfläche des distalen Endabschnitts das Stechmittel zumindest abschnittsweise kontaktiert, wodurch das Stechmittel zumindest abschnittsweise fixiert wird.

Durch die Erfindung wird nun ein Verfahren bereitgestellt, welches äußerst einfach eine Fixierung für das Stechmittel bereitstellt. Bei der Herstellung des Spritzenkörpers sind keine besonderen Modifikationen zu beachten. Insbesondere der distale Endabschnitt kann vorzugsweise mit einem einfachen Kanal ohne Anschlag für das Stechmittel hergestellt werden. Das Stechmittel kann durch die distale Öffnung in den Kanal eingeführt werden und wird dann durch den Kontakt mit der inneren Oberfläche des Formabschnitts fixiert. Unter einer Fixierung des Stechmittels ist eine Befestigung des Stechmittels zu verstehen, welche einer Abzugskraft, also der aufzuwendenden Kraft, die notwendig ist, um das Stechmittel aus dem Kanal heraus zu ziehen, von wenigstens 30N, bevorzugt wenigstens 60N weiter bevorzugt wenigstens 90 N standhält.

Vorzugsweise besteht der Spritzenkörper aus Borosilikatglas oder Quarzglas. Es wäre jedoch auch denkbar das Verfahren auf einen Spritzenkörper bestehend aus einem Kunststoff anzuwenden.

Vorzugsweise wird der formbare Zustand des distalen Endabschnitts durch Erwärmen erzeugt. Hierzu können verschiedenste Mittel verwendet werden, beispielsweise Brenner, Strahlungsquellen oder Laser. Bei Anwendung des Verfahrens bei einer Glasspritze würde die Temperatur des distalen Endabschnitts in einem Bereich zwischen 1000°C und 1200°C, bevorzugt bei 1100°C liegen.

Nach einer bevorzugten Ausführungsform umfasst die Herstellung des Spritzenkörpers folgende Schritte:
aa) Bereitstellen eines sich entlang einer axialen Richtung (X) erstreckenden hohlzylindrischen Glasrohlings mit zumindest einem offenen distalen Ende, wobei der Glasrohling ausgehend von dem offenen distalen Ende einen sich in axialer Richtung (X) erstreckenden Formabschnitt aufweist, der sich in einem formbaren Zustand befindet;
bb) Formen des Formabschnitts zu dem vorzugsweise konusförmigen distalen Endabschnitt des Spritzenkörpers mittels eines zweiten und eines dritten Formwerkzeugs;

Diese Herstellungsschritte aa) und bb), also das Formen des Formabschnitts zu dem distalen Endabschnitt, werden vor dem Schritt a), dem Bereitstellen eines Spritzenkörpers mit einem distalen Endabschnitt, durchgeführt.

Nach einer weiteren bevorzugten Ausführungsform umfasst Schritt bb) folgende Teilschritte:
(1) Bereitstellen des zweiten Formwerkzeugs, durch welches zumindest der Formabschnitt des hohlzylindrischen Glasrohlings verformbar ist;
(2) Bereitstellen eines dritten Formwerkzeugs, welches einen Formstift aufweist;
(3) Einführen des Formstifts durch das offene distale Ende des hohlzylindrischen Glasrohlings in dessen Formabschnitt;
(4) Formen des Formabschnitts durch das zweite Formwerkzeug derart, dass die innere Oberfläche des Formabschnitts an dem Formstift anliegt, wodurch der Formabschnitt den Kanal ausbildet;
(5) Entfernen des Formstifts aus dem Kanal.

Vorzugsweise erfolgt das Formen des distalen Endabschnitts in Schritt d) direkt im Anschluss nach der Herstellung des Spritzenkörpers. Der distale Endabschnitt würde somit nach dem Einführen des Stechmittels sich immer noch in einem ausreichend formbaren Zustand befinden, so dass die weitere Verformung in Schritt d) durchführbar ist.

Der Spritzenkörper kann aber auch zunächst einem weiteren Prozess zugeführt werden, in dem der distale Endabschnitt erwärmt wird, so dass dieser wieder in einen formbaren Zustand überführt wird. Dieser Prozess kann die bereits erwähnten Mittel wie Brenner, Strahlungsquelle oder Laser beinhalten.

Gemäß einer weiteren bevorzugten Ausführungsform ist das Stechmittel insbesondere in den Schritten b) und c) drehbar in einer Haltevorrichtung gelagert. Bevorzugt besteht das Stechmittel aus einem rostfreien Edelstahl. Gängige Kanülen haben spezielle sehr feine Schliffe, beispielsweise einen Einfachschliff, einen Facettenschliff oder einen V-Bevel-Schliff. Es ist dabei vorteilhaft wenn der Schliff eine bestimmte Position relativ zu dem Spritzenkörper einnimmt, beispielsweise für die weiteren Prozesse wie das Aufsetzen einer Schutzkappe. Auch für die Verwendung der Spritze ist eine Ausrichtung des Schliffes relativ zu einem Fingerflansch vorteilhaft. Demnach kann vorteilhafterweise eine Sensoreinrichtung die relative Orientierung des Stechmittels beziehungsweise des Schliffes an dem Stechmittel in der Halteeinrichtung zu dem Spritzenkörper detektieren. Durch drehbare Lagerung kann die Kanüle dann entsprechend ausgerichtet werden.

Nach einer weiteren bevorzugten Ausführungsform weist der distale Endabschnitt eine Länge (L) auf. Vorzugsweise findet das Formen in Schritt d) zumindest entlang eines Teilabschnitts der Länge L statt. Bevorzugt kontaktiert dadurch ein Kontaktabschnitt des Kanals beziehungsweise der inneren Oberfläche des distalen Endabschnitts das Stechmittel. Bevorzugt wird das Stechmittel dadurch zumindest rudimentär fixiert. Vorzugsweise wird das Stechmittel vollständig entlang seines Umfangs (U) von der inneren Oberfläche des distalen Endabschnitts kontaktiert.

Vorzugsweise erstreckt sich die Länge (L) des distalen Endabschnitts zwischen einem Hauptabschnitt des Spritzenkörpers, in welchem das Medium lagerbar ist, beziehungsweise einem Übergangsbereich zwischen dem Hauptabschnitt und dem distalen Endabschnitt und der distalen Öffnung durch welche das Stechmittel eingeführt wurde. Der Kontaktabschnitt des Kanals befindet sich vorzugsweise am zum Hauptabschnitt des Spritzenkörpers führenden Ende der Länge (L). Dadurch, dass das Stechmittel der inneren Oberfläche des distalen Endabschnitts entlang des ganzen Umfangs (U) des Stechmittels an diesem anliegt, wird nicht nur eine Fixierung des Stechmittels erreicht, es wird auch ein Eindringen von Kontaminierungen in den Hauptabschnitt des Spritzenkörpers verhindert. Vorzugsweise erstreckt sich der Kontaktabschnitt entlang 5% bis 90% der Länge (L) des distalen Endabschnitts, bevorzugt entlang 20% bis 80% der Länge (L) des distalen Endabschnitts, besonders bevorzugt entlang 30% bis 50% der Länge (L) des distalen Endabschnitts.

Nach einer weiteren bevorzugten Ausführungsform wird nach dem Formen in Schritt d) ein vorzugsweise organischer Klebstoff durch die distale Öffnung in einen distalen Abschnitt des Kanals eingebracht, wodurch das Stechmittel weiter an den distalen Endabschnitt fixiert wird. Unter einer Fixierung des Stechmittels ist eine Befestigung des Stechmittels zu verstehen, welche einer Abzugskraft, also der aufzuwendenden Kraft, die notwendig ist, um das Stechmittel aus dem Kanal heraus zu ziehen, von wenigstens 30N, bevorzugt wenigstens 60N weiter bevorzugt wenigstens 90 N standhält. Vorzugsweise wird ein Klebstoff auf Polymer- oder Epoxidbasis verwendet.

Der Klebstoff wird vorzugsweise durch die distale Öffnung eingebracht. Durch den bevorzugten Einsatz eines Initiators kann bei dessen Aktivierung bereits eine Vorhärtung des Klebstoffs erfolgen. Eine endgültige Aushärtung kann dann vorzugsweise in einem Aushärteprozess stattfinden. Die dazu geeigneten Mittel sind von dem verwendeten Klebstoff abhängig. Vorzugsweise könnte eine UV-Aushärtung oder eine thermische Aushärtung stattfinden.

Dadurch, dass das Stechmittel der inneren Oberfläche des distalen Endabschnitts entlang des ganzen Umfangs (U) des Stechmittels an diesem anliegt, wird nicht nur eine Fixierung des Stechmittels erreicht, es wird auch ein unerwünschtes Eindringen des Klebstoffs in den Hauptabschnitt des Spritzenkörpers verhindert.

Gemäß einer weiteren bevorzugten Ausführungsform findet das Formen in Schritt d) über die im Wesentlichen gesamte Länge (L) des distalen Endabschnitts statt. Bevorzugt kontaktiert dadurch ein Kontaktabschnitt des Kanals beziehungsweise der inneren Oberfläche des distalen Endabschnitts das Stechmittel. Vorzugsweise wird durch diese Kontaktierung das Stechmittel fixiert, so dass eine Abzugskraft von wenigstens 30 N ohne die Verwendung eines weiteren Klebstoffs erreicht wird. Vorzugsweise wird das Stechmittel vollständig entlang seines Umfangs (U) von der inneren Oberfläche des Formabschnitts kontaktiert.

Vorzugsweise erstreckt sich die Länge (L) des distalen Endabschnitts zwischen einem Hauptabschnitt des Spritzenkörpers in welchem das Medium lagerbar ist, beziehungsweise einem Übergangsbereich zwischen dem Hauptabschnitt und dem distalen Endabschnitt und der distalen Öffnung durch welche das Stechmittel eingeführt wurde. Der Kontaktabschnitt des Kanals befindet sich vorzugsweise am zum Hauptabschnitt des Spritzenkörpers führenden Ende der Länge (L) und erstreckt sich im Wesentlichen über die gesamte Länge (L). Dadurch, dass das Stechmittel der inneren Oberfläche des distalen Endabschnitts entlang des ganzen Umfangs (U) des Stechmittels an diesem anliegt und der Kontaktabschnitt sich im Wesentlichen über die gesamte Länge (L) erstreckt wird eine effektive Fixierung des Stechmittels erreicht. Auf die Verwendung eines Klebstoffs kann somit gänzlich verzichtet werden. Ferner wird durch das Anliegen der inneren Oberfläche des Formabschnitts entlang des ganzen Umfangs (U) des Stechmittels ein Eindringen von Kontaminierungen in den Hauptabschnitt des Spritzenkörpers verhindert.

Nach einer weiteren bevorzugten Ausführungsform umfassen das erste und/oder das zweite Formwerkzeug zwei voneinander beabstandete Formrollen. Vorzugsweise sind die Formrollen in einer ersten Position durch einen ersten Abstand beabstandet, wobei der distale Endabschnitt des Spritzenkörpers beziehungsweise des hohlzylindrischen Glasrohlings zwischen die Formrollen verlagerbar ist, wenn die Formrollen sich in der ersten Position befinden

Vorzugsweise sind die Formrollen des zweiten Formwerkzeugs zu einer zweiten Position verlagerbar, in welcher diese durch einen zweiten Abstand beabstandet sind, welcher kleiner ist als der erste Abstand. Bevorzugt ist in der zweiten Position durch die Formrollen eine Verformungskraft auf den Formabschnitt des hohlzylindrischen Glasrohlings beaufschlagbar, wodurch eine äußere Formgebung des Formabschnitts realisierbar ist. Vorteilhafterweise ist dabei eine innere Formgebung des Formabschnitts durch den Formstift des dritten Formwerkzeugs realisierbar.

Vorzugsweise weisen die Formrollen des ersten Formwerkzeugs eine Breite auf, die kleiner oder gleich der Länge (L) des distalen Endabschnitts sind. Durch Formrollen mit einer Breite, welche im Wesentlichen der Länge (L) des distalen Endabschnitts entspricht, kann ein Kontaktabschnitt geformt werden, welcher im Wesentlichen der Länge (L) entspricht. Durch Formrollen, deren Breite kleiner als die Länge (L) des distalen Endabschnitts ist, kann ein entsprechend kleinerer Kontaktabschnitt geformt werden.

Bevorzugt sind die Formrollen des ersten Formwerkzeugs zu einer zweiten Position verlagerbar, in welcher diese durch einen zweiten Abstand beabstandet sind, welcher kleiner ist als der erste Abstand. Bevorzugt ist in der zweiten Position durch die Formrollen eine Verformungskraft auf den distalen Endabschnitt beaufschlagbar, wodurch eine Formgebung des distalen Endabschnitts realisierbar ist und die innere Oberfläche des distalen Endabschnitts an das Stechmittel gedrückt wird.

Nach einer weiteren bevorzugten Ausführungsform erfolgt das erstmalige Formen des distalen Endabschnitts in Schritt bb) und das weitere Formen des distalen Endabschnitts in Schritt d) durch das erste Formwerkzeug. Demnach würde nach der Formgebung des Formabschnitts zu dem distalen Endabschnitt der Formstift aus dem Kanal entfernt und das dritte Formwerkzeug entfernt. Anschließend wird die Haltevorrichtung mit dem Stechmittel derart angeordnet, dass ein mittiges Einführen des Stechmittels, relativ zur axialen Mittelachse des Spritzenkörpers möglich ist. Die weitere Formgebung kann dann durch die gleichen Formrollen des ersten Formwerkzeugs erfolgen. Es wäre denkbar die Formrollen derart auszugestalten, dass deren Breite variiert werden kann. Demnach kann der Kontaktabschnitt entsprechend ausgebildet werden.

Bei der Verwendung zweier Formwerkzeuge (erstes und zweites Formwerkzeug) wird nach dem Entfernen des Formstifts der Glasrohling aus dem Zwischenraum zwischen den Formrollen des zweiten Formwerkzeugs entfernt und in dem Zwischenraum des ersten Formwerkzeugs platziert. Dies hat zum Vorteil, dass die Haltevorrichtung für das Stechmittel und das dritte Formwerkzeug mit dem Formstift den Formrollen des ersten beziehungsweise des zweiten Formwerkzeugs zugeordnet sind.

Vorzugsweise ist der hohlzylindrische Glasrohling an einer Haltevorrichtung derart angeordnet, dass dieser rotierbar ist, wobei die Rotationsachse eine axiale Mittelachse des hohlzylindrischen Glasrohlings ist. Bevorzugt ist das dritte Formwerkzeug mit dem Formstift mittig zu dieser axialen Mittelachse angeordnet.

Bevorzugt bleiben beim Andrücken der inneren Oberfläche an das Stechmittel die Formrollen des ersten Formwerkzeugs an einer festen Position. Um nun eine Kontaktierung des Stechmittels über den gesamten Umfang (U) des Stechmittels zu erreichen, kann der Spritzenkörper vorteilhafterweise durch die Haltevorrichtung entsprechend gedreht werden. Das Stechmittel kann bevorzugt während dieses Prozesses mittels der drehbaren Haltevorrichtung entsprechend synchron mit der Haltevorrichtung des Spritzenkörpers gedreht werden.

Alternativ kann vorteilhafterweise nach einem ersten punktuellen Kontaktieren das Stechmittel soweit von seiner Halterung gelöst werden, so dass es der Rotation des Spritzenkörpers folgen kann. Durch den erstmaligen punktuellen Kontakt ist bereits eine ausreichende Kraftschlüssigkeit vorhanden, um dies zu gewährleisten. Die Halterung fungiert somit vorzugsweise nur noch als eine Führung für die Drehung des Stechmittels.

Während die Formwerkzeuge auf der Außenseite der Röhre unkritisch sind, da ein eventuell entstehender Abrieb nicht mit dem Füllgut in Kontakt kommt, sind alle Werkzeuge, welche innerhalb des Glasrohlings Anwendung finden kritisch zu betrachten. Die derzeit im Stand der Technik eingesetzten Werkzeuge bestehen nahezu zu 100% aus Wolfram. Dieses Material eignet sich zwar gut für die außen anliegenden Werkzeuge, für Werkzeuge, welche innerhalb des Glasbehältnisses eingesetzt werden ist es allerdings weniger geeignet. Der Formstift für den Kanal des Spritzenkonus ist sehr klein und heizt sich durch die geringe Wärmekapazität stark auf, so dass Oxide entstehen, die zu Verschleiß und Abrieb führen. Der Abrieb des Formstifts liegt in einer Größenordnung, so dass der Formstift in der Regel nach ca. 1 Stunde Betrieb ausgetauscht werden muss. Dieser Abrieb schlägt sich in dem Kanal nieder, wird durch das Füllgut später absorbiert und führt zu Beeinträchtigung von Wirksamkeit, Verträglichkeit.

Demnach besteht der Formstift vorzugsweise aus einem nichtmetallischen Material, beispielsweise aus einer technischen Keramik, einem keramikartigen Material oder glasartigen Kohlenstoff. Keramische Werkstoffe sind polykristallin, anorganisch und nichtmetallisch. Sie werden in der Regel bei Raumtemperatur aus einer Rohmasse geformt und erhalten ihre typischen Werkstoffeigenschaften durch einen Sintervorgang, der bei hohen Temperaturen stattfindet. Der Ausdruck technische Keramik ist der Oberbegriff für keramische Werkstoffe und daraus hergestellte Produkten für technische Anwendungen. Technische Keramiken sind weiterhin unterteilt in Silikatkeramiken, Oxidkeramiken und Nichtoxidkeramiken. Die Nichtoxidkeramiken werden weiterhin in carbidische und nitridische Nichtoxidkeramiken unterschieden.

Besonders bevorzugt besteht der Formstift aus Siliziumnitrid (Si₃N₄) oder glasartigem Kohlenstoff. Weitere bevorzugte Materialen sind Zirkoniumoxid und Zirkonium verstärktes Aluminiumoxid (ZTA). Siliziumnitrid ist eine Nichtoxidkeramik und weist eine hohe Bruchzähigkeit und einen niedrigen Wärmeausdehnungskoeffizienten auf. Es zeigte eine Dauergebrauchstemperatur von 1300°C und eine vergleichsweise sehr gute Temperaturwechsel-Belastbarkeit. Glasartiger Kohlenstoff oder Glaskohlenstoff zeigt eine besonders geringe Neigung an dem Glasrohling während der Formung zu kleben, jedoch liegt die Dauergebrauchstemperatur lediglich bei 600 °C. Allerdings kann diese Dauergebrauchstemperatur durch Verwendung einer Schutzgasatmosphäre auf über 2000 °C erhöht werden.

Nach einer weiteren bevorzugten Ausführungsform wird der Formstift durch eine Kühleinrichtung gekühlt, wodurch die Standzeit des Formstifts erhöht wird.

Vorteilhafterweise wird nach dem Schritt d) der mit dem Stechmittel versehene Spritzenkörper abgekühlt und/oder einem Sterilisierungsprozess zugeführt. Die Sterilisation kann durch Flüssigkeiten oder Dämpfe erfolgen. Denkbar wäre auch eine trockene Sterilisation durch Erhitzen. Ferner kann auf die innere Oberfläche des Hauptabschnitts ein Gleitmittel, beispielsweise Silikonöl aufgebracht werden. Die Spritzen können dann mit einem Nadelschutz versehen werden und in Trays oder Kartons verpackt werden. In der Regel werden die Spritzen dann bei einem Pharmahersteller befüllt. Anschließend wird dann der Kolben mit dem Stopfen eingesetzt.

Die Aufgabe wird weiterhin durch eine Spritze mit einem Stechmittel gelöst, welche durch ein Verfahren nach einem der vorhergehenden Ansprüche hergestellt worden ist.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig.1: Eine schematische Anordnung zum Verfahren zur Herstellung eines Spritzenkörpers;
- Fig.2: eine weitere schematische Anordnung zum Verfahren zur Herstellung eines Spritzenkörpers;
- Fig.3: eine Schnittdarstellung eines Spritzenkörpers und eines Stechmittels;
- Fig.3a: eine Darstellung eines Stechmittels;
- Fig.4: eine Schnittdarstellung des Spritzenkörpers mit einem Stechmittel;
- Fig.5: eine schematische Anordnung zum Verfahren zur Herstellung einer Spritze mit einem Stechmittel;
- Fig.6: eine weitere schematische Anordnung zum Verfahren zur Herstellung einer Spritze mit einem Stechmittel;
- Fig.7: eine schematische Anordnung zu einem weiteren Verfahren zur Herstellung einer Spritze mit einem Stechmittel;
- Fig.8: eine weitere schematische Anordnung zu einem weiteren Verfahren zur Herstellung einer Spritze mit einem Stechmittel.

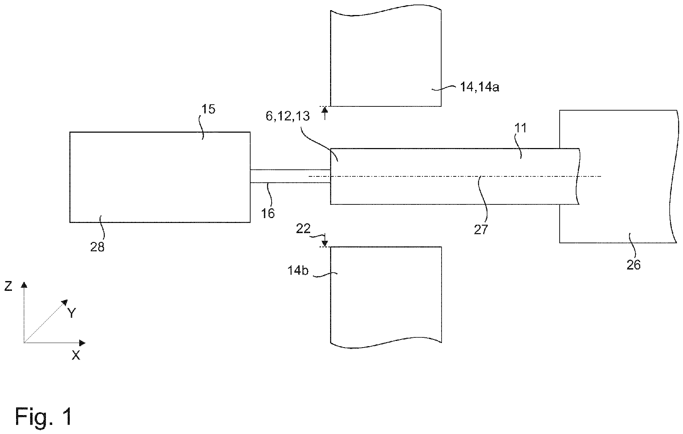

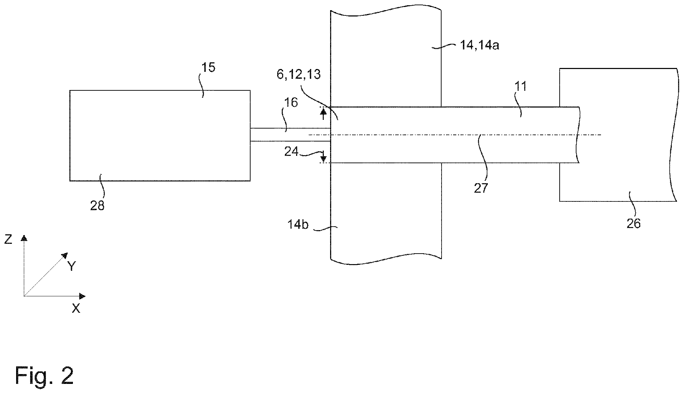

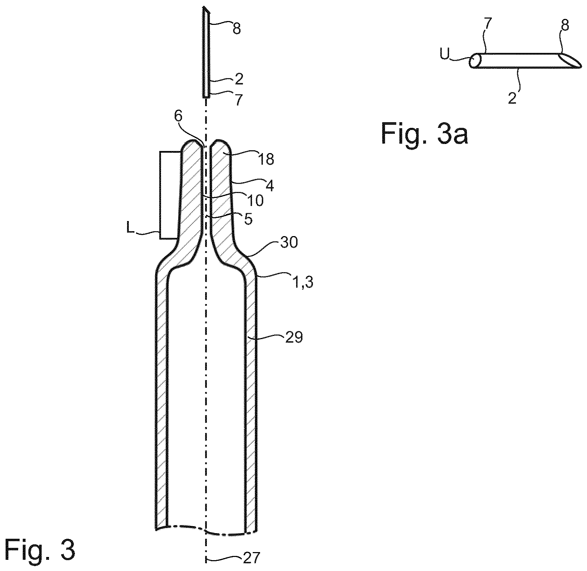

Die Figuren 1 und 2 zeigen eine schematische Anordnung für ein Verfahren zur Herstellung eines Spritzenkörpers (3) mit einem distalen Endabschnitt(4). Ein solcher Spritzenkörper (3) ist in Fig. 3 dargestellt und wird aus einem sich entlang einer axialen Richtung (X) erstreckenden hohlzylindrischen Glasrohling (11) mit zumindest einem offenen Ende (6) hergestellt. Dieser Glasrohling (11) weist ausgehend von dem offenen Ende (6) einen sich in axialer Richtung (X) erstreckenden Formabschnitt (13) auf, der sich in einem formbaren Zustand befindet.

Der formbare Zustand wird in der Regel durch Erwärmen des Glases realisiert, wobei die Temperatur in einem Bereich zwischen 1000 °C und 1200 °C, bevorzugt bei etwa 1100 °C liegt. Der hohlzylindrische Glasrohling (11) ist an einer Haltevorrichtung (26) derart angeordnet ist, dass dieser rotierbar ist, wobei die Rotationsachse eine axiale Mittelachse (27) des hohlzylindrischen Glasrohlings (11) ist. Durch eine Rotation des Glasrohlings wird eine gleichmäßige Verformung dessen gewährleistet.

In den Figuren 1 und 2 ist weiterhin ein zweites Formwerkzeug (14) gezeigt, durch welches zumindest der Formabschnitt (13) des hohlzylindrischen Glasrohlings (11) verformt wird. Der Formabschnitt (13) wird durch das zweite Formwerkzeug (14) derart verformt, dass eine innere Oberfläche (10) des Formabschnitts (13) an einem Formstift (16) anliegt, wodurch der Formabschnitt (13) einen Kanal (5) ausbildet. Das zweite Formwerkzeug (14) umfasst dabei zwei voneinander beabstandete Formrollen (14a, 14b). In der in Figur 1 gezeigten Konfiguration befinden sich die Formrollen (14a, 14b) in einer ersten Position, in welcher sie durch einen ersten Abstand (22) beabstandet sind. Befinden sich die Formrollen (14a, 14b) in dieser ersten Position wird zumindest der Formabschnitt (13) des hohlzylindrischen Glasrohlings (11) zwischen die Formrollen (14a, 14b) verlagert. Dies kann beispielsweise über eine entsprechende Transportvorrichtung erfolgen, welche Glasrohlinge der Vorrichtung zuführt und nach der Bearbeitung zu dem nächsten Fertigungsschritt transportiert.

Ferner ist in den Figuren 1 und 2 weiterhin ein drittes Formwerkzeug (15) gezeigt, welches einen Formstift (16) aufweist. Dieser Formstift (16) besteht vorzugsweise aus einem nichtmetallischen Material, bevorzugt aus einer technischen Keramik oder einem keramikartigen Material und besonders bevorzugt aus Siliziumnitrid (Si₃N₄) oder glasartigem Kohlenstoff. Der Formstift (16) ist durch eine Fixiereinrichtung (28) des dritten Formwerkzeugs (15) fixiert und durch das offene Ende (12) des hohlzylindrischen Glasrohlings (11) in dessen Formabschnitt (13) entlang der axialen Richtung (X) verlagert.

In der in Figur 2 gezeigten Konfiguration befinden sich die Formrollen (14a, 14b) in einer zweiten Position. In dieser zweiten Position sind die Formrollen (14a, 14b) durch einen zweiten Abstand (24) voneinander beabstandet. Dieser zweite Abstand (24) ist kleiner als der erste Abstand (22). Die Formrollen liegen an dem Formabschnitt (13) des Glasrohlings (11) an, wodurch eine Verformungskraft auf den Formabschnitt (13) beaufschlagt wird. Somit wird eine äußere Formgebung des Formabschnitts (13) realisiert. Die innere Formgebung des Formabschnitts (13) beziehungsweise die Formgebung des Kanals (5) wird durch Gegenhalten des Formstifts (16) realisiert. Die Form des Kanals (5) ist dabei von der Form des Formstifts (16) abhängig. Um die Einsatzzeiten des Formstifts zu erhöhen, ist es vorteilhaft, wenn dieser durch eine Kühleinrichtung gekühlt wird.

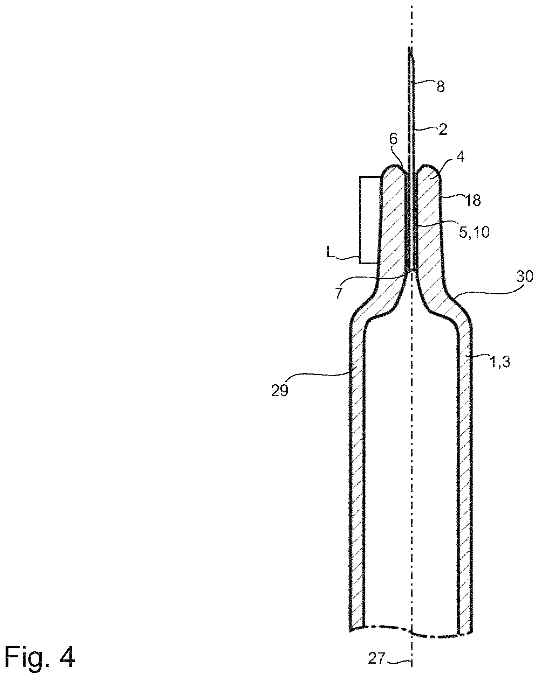

In Figur 3 ist ein derart hergestellter Spritzenkörper (3) gezeigt. Figur 4 zeigt einen Spritzenkörper mit einem darin befestigten Stechmittel (2). Der Spritzenkörper ist hohlzylindrisch ausgestaltet und weist einen Hauptabschnitt (29) auf, in dessen Hohlraum das zu applizierende Medium gelagert wird. Der Außendurchmesser des Hauptabschnitts (29) ist größer als der Außendurchmesser des distalen Endabschnitts (4), wobei sich zwischen dem Hauptabschnitt (29) und dem distalen Endabschnitt ein Übergangsbereich (30) befindet. Ebenso ist der Innendurchmesser des distalen Endabschnitts (4) kleiner als der Innendurchmesser des Hauptabschnitts (29). Der innere Kanal (5) des distalen Endabschnitts (4) ist dabei mit dem Hohlraum des Hauptabschnitts (29) verbunden.

Darüber hinaus ist der distale Endabschnitt(4) in der Regel konisch ausgebildet. Derartige Spritzenkörper sind oft an deren proximalen Ende mit einem Fingerflansch ausgestattet (nicht gezeigt in den Figuren). Ferner ist nach dem Befüllen der Spritze mit dem entsprechenden Medium ein Kolben mit einem Stopfen in die proximale Öffnung des Spritzenkörpers (3) eingeführt (Ebenso nicht in den Figuren gezeigt).

In Figur 3a ist ein Stechmittel (2) gezeigt. Das Stechmittel ist hohlzylindrig ausgestaltet und weist einen proximalen Abschnitt (7) sowie einen distalen Abschnitt (8) auf. Am distalen Anschnitt (8) ist ein entsprechender Schliff angebracht. Ferner weist das Stechmittel einen Umfang (U) auf.

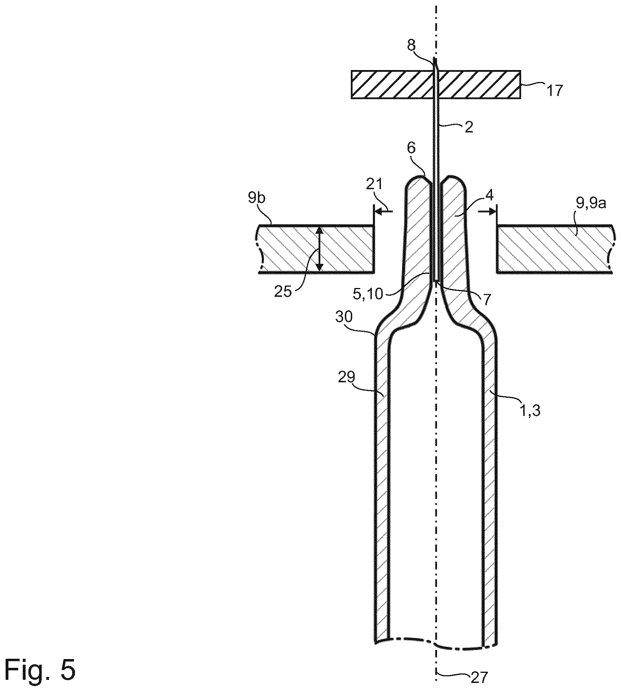

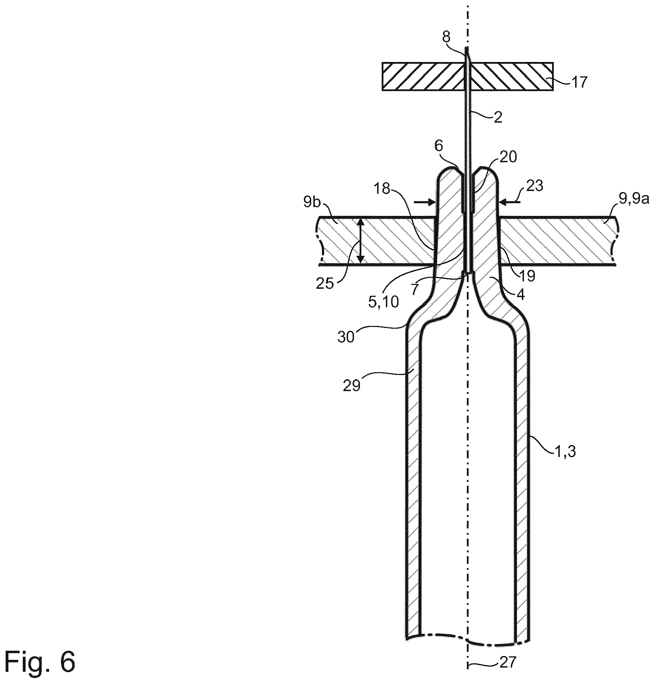

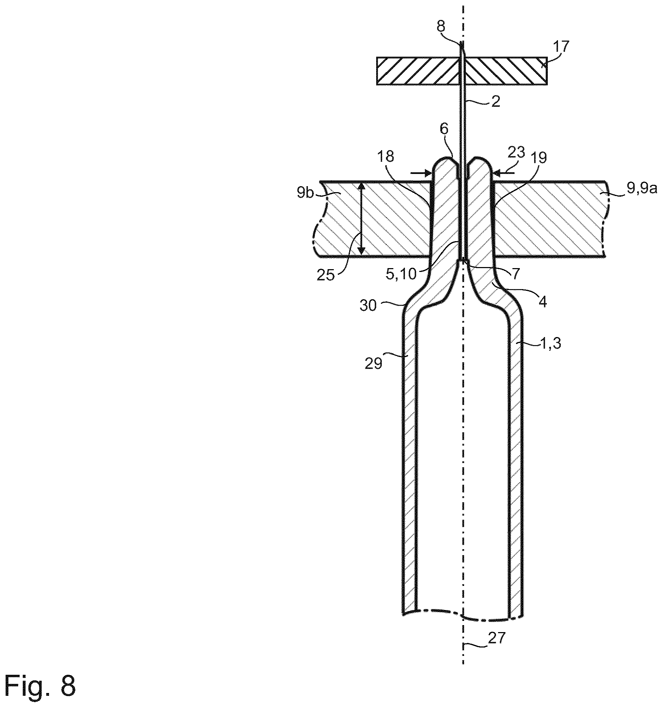

Das Verfahren zur Herstellung einer Spritze (1) mit einem Stechmittel (2) ist in den Figuren 5 bis 8 gezeigt umfassend folgende Schritte:
a) Bereitstellen eines Spritzenkörpers (3) mit einem distalen Endabschnitt (4), welcher einen inneren Kanal (5), der in einer distalen Öffnung (6) mündet, aufweist, wobei der distale Endabschnitt (4) sich in einem formbaren Zustand befindet;
b) Bereitstellen eines Stechmittels (2);
c) Einführen eines proximalen Abschnitts (7) des Stechmittels (2) durch die distale Öffnung (6) in den inneren Kanal (5) des distalen Endabschnitts (4);
d) Formen des distalen Endabschnitts (4) mittels eines ersten Formwerkzeugs (9) derart, dass eine innere Oberfläche (10) des distalen Endabschnitts (4) das Stechmittel (2) zumindest abschnittsweise kontaktiert, wodurch das Stechmittel (2) zumindest abschnittsweise fixiert.

Das erste Formwerkzeug (9) umfasst zwei voneinander beabstandete Formrollen (9a, 9b), wobei die Formrollen (9a, 9b) in einer ersten Position durch einen ersten Abstand (21) beabstandet sind, wobei der distale Endabschnitt (4) zwischen die Formrollen (9a, 9b) verlagerbar ist, wenn die Formrollen (9a, 9b) sich in der ersten Position befinden

Nach der Ausformung des Spritzenkörpers (3) aus dem hohlzylindrischen Glasrohling (11) wird nach dem Entfernen des Formstifts (16) aus dem Kanal (5) der Spritzenkörper (3) aus dem Zwischenraum zwischen den Formrollen (14a, 14b) des zweiten Formwerkzeugs (14) entfernt und in einem Zwischenraum zwischen den Formrollen (9a, 9b) des ersten Formwerkzeugs (9) platziert.

Das Formen des distalen Endabschnitts (4) in Schritt d) kann direkt im Anschluss nach der Herstellung des Spritzenkörpers (3) erfolgen. Alternativ, falls der distale Endabschnitt sich nicht mehr in einem ausreichend formbaren Zustand befindet, kann der Spritzenkörper (3) zunächst einem weiteren Prozess zugeführt wird, in dem der distale Endabschnitt (4) erwärmt wird, so dass dieser wieder in einen formbaren Zustand überführt wird.

Das Stechmittel (2) wird zunächst mittels Haltevorrichtung (17) entlang der axialen Mittelachse (27) des Spritzenkörpers (3) in den Kanal (5) des distalen Endabschnitts (4) eingeführt. Nachdem das Stechmittel (2) in den Kanal (5) eingeführt worden ist, werden die Formrollen (9a, 9b) in eine zweiten Position verlagert, in welcher diese durch einen zweiten Abstand (23) beabstandet sind, der kleiner ist als der erste Abstand (21). In dieser Position wird durch die Formrollen (9a, 9b) eine Verformungskraft auf den distalen Endabschnitt (4) ausgeübt, wodurch eine Formgebung des distalen Endabschnitts (4) realisierbar ist und die innere Oberfläche des distalen Endabschnitts (4) an das Stechmittel (2) gedrückt wird.

Das Stechmittel (2), insbesondere in den Schritten b) und c), ist dabei drehbar in einer Haltevorrichtung (17) gelagert. Durch die drehbare Lagerung kann die Ausrichtung des Stechmittels (2), insbesondere der Schliff des Stechmittels (2), relativ zu dem Spritzenkörper ausgerichtet werden.

Der hohlzylindrische Glasrohling ist an der Haltevorrichtung (26) derart angeordnet, dass dieser rotierbar ist, wobei die Rotationsachse eine axiale Mittelachse des hohlzylindrischen Glasrohlings beziehungsweise des Spritzenkörpers ist. Bevorzugt ist das dritte Formwerkzeug mit dem Formstift mittig zu dieser axialen Mittelachse angeordnet.

Beim Andrücken der inneren Oberfläche (10) an das Stechmittel (2) bleiben die Formrollen (9a, 9b) an einer festen Position. Um nun eine Kontaktierung des Stechmittels über den gesamten Umfang (U) des Stechmittels (2) zu erreichen, wird der Spritzenkörper (3) durch die Haltevorrichtung (26) entsprechend gedreht. Das Stechmittel (2) kann nun während dieses Prozesses, mittels der drehbaren Haltevorrichtung (17), entsprechend synchron mit der Haltevorrichtung (26) des Spritzenkörpers (3) gedreht werden. Alternativ kann nach einem ersten punktuellen Kontaktieren das Stechmittel (2) soweit von seiner Halterung gelöst werden, dass es der Rotation des Spritzenkörpers folgen kann. Durch den erstmaligen punktuellen Kontakt ist bereits eine ausreichende Kraftschlüssigkeit vorhanden, um dies zu gewährleisten. Die Halterung (17) fungiert somit nur noch als eine Führung für die Drehung des Stechmittels (2).

Es wäre auch denkbar, dass sowohl die Verformung des Formabschnitts (13) des hohlzylindrischen Glasrohlings (11) als auch die weitere Verformung des distalen Endabschnitts (4) durch das gleiche erste Formwerkzeug (9) erfolgt. Hierzu muss nach der Formung des distalen Endabschnitts (4) aus dem Formabschnitt (13) des hohlzylindrischen Glasrohlings (11) der Formstift (16) aus dem Kanal (5) entfernt werden und dann das dritte Formwerkzeug (15) von den Formrollen (9a, 9b) entfernt werden. Anschließend wird das Stechmittel (2) mittig entlang der axialen Mittelachse (27) des Spritzenkörpers (3) mittels der Haltevorrichtung (17) eingeführt.

Der distale Endabschnitt (4) weist eine Länge (L) auf. Nach einer Ausführungsform findet das Formen in Schritt d) entlang eines Teilabschnitts (18) der Länge (L) statt. Dadurch kontaktiert ein Kontaktabschnitt (19) des Kanals beziehungsweise der inneren Oberfläche (10) des distalen Endabschnitts (4) das Stechmittel (2). Die Länge (L) des distalen Endabschnitts (4) erstreckt sich zwischen dem Hauptabschnitt (29) des Spritzenkörpers (3), in dessen Hohlraum das Medium lagerbar ist, beziehungsweise dem Übergangsbereich (30) zwischen dem Hauptabschnitt (29) und dem distalen Endabschnitt (4), und der distalen Öffnung (6) durch welche das Stechmittel (2) eingeführt wurde.

Der Kontaktabschnitt (19) des Kanals (5) erstreckt sich ausgehend von dem Übergangsbereich (30) entlang eines Teilabschnitts (18) der Länge (L). Der Kontakt in dem Kontaktabschnitt (19) findet entlang des gesamten Umfangs (U) des Stechmittels (2) statt. Das Stechmittel (2) wird von dem Kontaktabschnitt also gänzlich entlang seines Umfangs (U) umschlossen. Dadurch wird das Stechmittel (2) zum einen zumindest rudimentär fixiert, zum anderen wird der Kanal (5) rund um das Stechmittel (2) verschlossen. Es können somit keine Kontaminierungen durch den Kanal (5) in den Hohlraum des Hauptabschnitts (29) des Spritzenkörpers (3) gelangen.

Vorzugsweise erstreckt sich der Kontaktabschnitt (19) entlang 5 % bis 90 % der Länge (L) des distalen Endabschnitts (4), bevorzugt entlang 20 % bis 80 % der Länge (L) des distalen Endabschnitts (4), besonders bevorzugt entlang 30 % bis 50 % der Länge (L) des distalen Endabschnitts (4).

Offensichtlich müssen die Formrollen (9a, 9b) des ersten Formwerkzeugs (9) dabei eine Breite (25) aufweisen, die kleiner der Länge (L) des distalen Endabschnitts (4) sind, beziehungsweise der Länge des Kontaktabschnitts (19) entsprechen. Dies ist in den Figuren 5 bis 6 gezeigt. Für den Fall, dass sowohl die Verformung des Formabschnitts (13) des hohlzylindrischen Glasrohlings (11) als auch die weitere Verformung des distalen Endabschnitts (4) durch das gleiche erste Formwerkzeug (9) erfolgt, müssten die Formrollen (9a, 9b) eine einstellbare Breite aufweisen.

Der distale Abschnitt (20) des Kanals (5) erstreckt sich zwischen dem Kontaktabschnitt (19) des Kanals (5) und der distalen Öffnung (6). In diesem Bereich liegt die innere Oberfläche (10) des distalen Endabschnitts (4) nicht an dem Stechmittel (2) an.

In diesem distalen Abschnitt (20) wird nach dem Formen in Schritt d) ein vorzugsweise organischer Klebstoff durch die distale Öffnung (6) eingebracht, wodurch das Stechmittel (2) weiter in dem Kanal (5) fixiert wird. Durch die Verklebung wird eine Abzugskraft von wenigstens 30 N, bevorzugt wenigstens 60 N weiter bevorzugt wenigstens 90 N erreicht. Unter einer Abzugskraft wird eine Kraft verstanden, welche benötigt wird um das Stechmittel (2) aus dem distalen Endabschnitt (4) heraus zu ziehen. Vorzugsweise wird ein Klebstoff auf Polymer- oder Epoxidbasis verwendet.

Nach einer weiteren Ausführungsform findet das Formen in Schritt d) über die im Wesentlichen gesamte Länge (L) des distalen Endabschnitts (4) statt. Der Kontaktabschnitt (19) des Kanals (5) beziehungsweise die innere Oberfläche (10) des distalen Endabschnitts (4) kontaktiert das Stechmittel (2) nun über die im Wesentlichen gesamte Länge (L) des distalen Endabschnitts (4). Der Kontaktabschnitt (19) des Kanals (5) erstreckt sich ausgehend von dem Übergangsbereich (30) entlang der im Wesentlichen gesamten Länge (L).

Der Kontakt in dem Kontaktabschnitt (19) findet entlang des gesamten Umfangs (U) des Stechmittels (2) statt. Das Stechmittel (2) wird von dem Kontaktabschnitt also gänzlich entlang ihres Umfangs (U) umschlossen. Dadurch wird das Stechmittel (2) zum einen fixiert, zum anderen wird der Kanal (5) rund um das Stechmittel (2) verschlossen. Es können somit keine Kontaminierungen durch den Kanal (5) in den Hohlraum des Hauptabschnitts (29) des Spritzenkörpers (3) gelangen. Durch die größere Kontaktfläche zwischen dem Stechmittel (2) und dem Kontaktabschnitt (19) kann eine Abzugskraft von wenigstens 30 N ohne die Verwendung eines weiteren Klebstoffs erreicht werden.

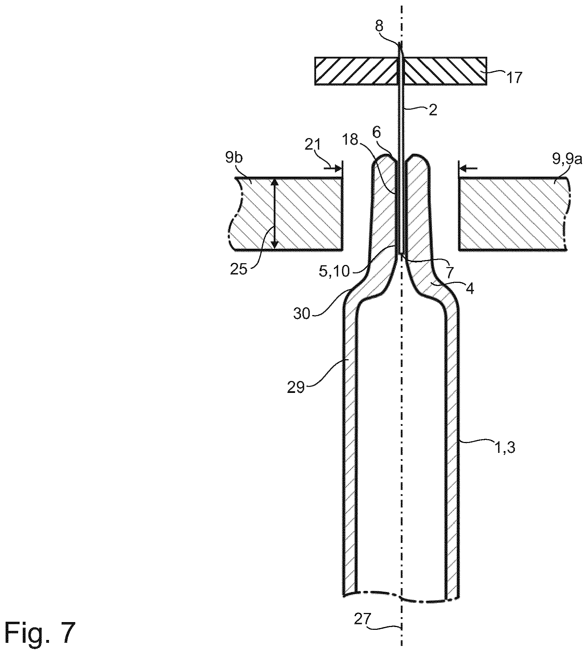

Offensichtlich müssen die Formrollen (9a, 9b) des ersten Formwerkzeugs (9) nun eine Breite (25) aufweisen, die im Wesentlichen gleich der Länge (L) des distalen Endabschnitts (4) ist. Dies ist in den Figuren 7 bis 8 gezeigt.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Spritze
- 2: Stechmittel
- 3: Spritzenkörper
- 4: distaler Endabschnitt des Spritzenkörpers
- 5: Kanal
- 6: distale Öffnung, offenes distales Ende
- 7: proximaler Abschnitt des Stechmittels
- 8: distaler Abschnitt des Stechmittels
- 9: erstes Formwerkzeug
- 9a, 9b: Formrollen des ersten Formwerkzeugs
- 10: innere Oberfläche des distalen Endabschnitts
- 11: hohlzylindrischer Glasrohling
- 12: offenes distales Ende des hohlzylindrischen Glasrohlings
- 13: Formabschnitt
- 14: zweites Formwerkzeug
- 14a, 14b: Formrollen des zweiten Formwerkzeugs
- 15: drittes Formwerkzeug
- 16: Formstift
- 17: Haltevorrichtung
- 18: Teilabschnitt der Länge (L)
- 19: Kontaktabschnitt des Kanals
- 20: distaler Abschnitt des Kanals
- 21: erster Abstand der Formrollen des ersten Formwerkzeugs
- 22: erster Abstand der Formrollen des zweiten Formwerkzeugs
- 23: zweiter Abstand der Formrollen des ersten Formwerkzeugs
- 24: zweiter Abstand der Formrollen des zweiten Formwerkzeugs
- 25: Breite der Formrollen des ersten Formwerkzeugs
- 26: Haltevorrichtung für den hohlzylindrischen Glasrohling
- 27: axiale Mittelachse des hohlzylindrischen Glasrohlings beziehungsweise des Spritzenkörpers
- 28: Fixiereinrichtung des dritten Formwerkzeugs
- 29: Hauptabschnitt des Spritzenkörpers
- 30: Übergangsbereich
- L: Länge des distalen Endabschnitts
- X: axiale Richtung
- U: Umfang des Stechmittels

## Patentansprüche

1. Verfahren zur Herstellung einer Spritze (1) mit einem Stechmittel (2) umfassend folgende Schritte:
a) Bereitstellen eines Spritzenkörpers (3) mit einem distalen Endabschnitt (4), welcher einen inneren Kanal (5), der in einer distalen Öffnung (6) mündet, aufweist, wobei der distale Endabschnitt (4) sich in einem formbaren Zustand befindet;
b) Bereitstellen eines Stechmittels (2);
c) Einführen eines proximalen Abschnitts (7) des Stechmittels (2) durch die distale Öffnung (6) in den inneren Kanal (5) des distalen Endabschnitts (4);
d) Formen des distalen Endabschnitts (4) mittels eines ersten Formwerkzeugs (9) derart, dass eine innere Oberfläche (10) des distalen Endabschnitts (4) das Stechmittel (2) zumindest abschnittsweise kontaktiert, wodurch das Stechmittel (2) zumindest abschnittsweise fixiert wird.

2. Verfahren nach Anspruch 1,
**dadurch gekennzeichnet, dass**
die Herstellung des Spritzenkörpers (3) folgende Schritte umfasst:
aa) Bereitstellen eines sich entlang einer axialen Richtung (X) erstreckenden hohlzylindrischen Glasrohlings (11) mit zumindest einem offenen distalen Ende (12), wobei der Glasrohling (11) ausgehend von dem offenen distalen Ende (12) einen sich in axialer Richtung (X) erstreckenden Formabschnitt (13) aufweist, der sich in einem formbaren Zustand befindet;
bb) Formen des Formabschnitts (13) zu dem vorzugsweise konusförmigen distalen Endabschnitt (4) des Spritzenkörpers (3) mittels eines zweiten (14) und eines dritten Formwerkzeugs (15);

3. Verfahren nach Anspruch 2,
**dadurch gekennzeichnet, dass**
Schritt bb) folgende Teilschritte umfasst:
(1) Bereitstellen des zweiten Formwerkzeugs (14), durch welches zumindest der Formabschnitt (13) des hohlzylindrischen Glasrohlings (11) verformbar ist;
(2) Bereitstellen eines dritten Formwerkzeugs (15), welches einen Formstift (16) aufweist;
(3) Einführen des Formstifts (16) durch das offene distale Ende (12) des hohlzylindrischen Glasrohlings (11) in dessen Formabschnitt (13);
(4) Formen des Formabschnitts (13) durch das zweite Formwerkzeug (14) derart, dass die innere Oberfläche (10) des Formabschnitts (13) an dem Formstift (16) anliegt, wodurch der Formabschnitt (13) den Kanal (5) ausbildet;
(5) Entfernen des Formstifts (16) aus dem Kanal (5).

4. Verfahren nach einem der vorhergehenden Ansprüche,
**dadurch gekennzeichnet, dass**
das Formen des distalen Endabschnitts (4) in Schritt d) direkt im Anschluss nach der Herstellung des Spritzenkörpers (3) erfolgt oder der Spritzenkörper (3) zunächst einem weiteren Prozess zugeführt wird, in dem der distale Endabschnitt (4) erwärmt wird, so dass dieser in einen formbaren Zustand überführt wird.

5. Verfahren nach einem der vorhergehenden Ansprüche,
**dadurch gekennzeichnet, dass**
das Stechmittel (2) insbesondere in den Schritten b) und c) drehbar in einer Haltevorrichtung (17) gelagert wird.

6. Verfahren nach einem der vorhergehenden Ansprüche,
**dadurch gekennzeichnet, dass**
der distale Endabschnitt (4) eine Länge (L) aufweist, wobei das Formen in Schritt d) entlang eines Teilabschnitts (18) der Länge (L) stattfindet, wodurch ein Kontaktabschnitt (19) des Kanals beziehungsweise der inneren Oberfläche (10) des distalen Endabschnitts (4) das Stechmittel (2) kontaktiert und zumindest rudimentär fixiert, wobei das Stechmittel (2) vollständig entlang seines Umfangs (U) von der inneren Oberfläche (10) des distalen Endabschnitts (4) kontaktiert wird.

7. Verfahren nach Anspruch 6,
**dadurch gekennzeichnet, dass**
nach dem Formen in Schritt d) ein vorzugsweise organischer Klebstoff durch die distale Öffnung (6) in einen distalen Abschnitt (20) des Kanals (5) eingebracht wird, wodurch das Stechmittel (2) in dem distalen Endabschnitt (4) fixiert wird, so dass eine Abzugskraft von wenigstens 30 N ohne die Verwendung eines weiteren Klebstoffs erreicht wird, wobei der distale Abschnitt (20) des Kanals (5) durch die distale Öffnung (6) und den Kontaktabschnitt (19) des Kanals (5) begrenzt wird.

8. Verfahren nach einem der vorhergehenden Ansprüche 1 bis 5,
**dadurch gekennzeichnet, dass**
das Formen in Schritt d) über die im Wesentlichen gesamte Länge (L) des distalen Endabschnitts (4) stattfindet, wodurch ein Kontaktabschnitt (19) des Kanals (5) beziehungsweise der inneren Oberfläche (10) des distalen Endabschnitts (4) das Stechmittel (2) kontaktiert und fixiert, so dass eine Abzugskraft von wenigstens 30 N ohne die Verwendung eines weiteren Klebstoffs erreicht wird, wobei das Stechmittel (2) vollständig entlang seines Umfangs (U) von der inneren Oberfläche (10) des distalen Endabschnitts (4) kontaktiert wird.

9. Verfahren nach einem der vorhergehenden Ansprüche,
**dadurch gekennzeichnet, dass**
das erste (9) und/oder das zweite Formwerkzeug (14) zwei voneinander beabstandete Formrollen (9a, 9b, 14a, 14b) umfassen, wobei die Formrollen (9a, 9b, 14a, 14b) in einer ersten Position durch einen ersten Abstand (21, 22) beabstandet sind, wobei der distale Endabschnitt (4) zwischen die Formrollen (9a, 9b, 14a, 14b) verlagerbar ist, wenn die Formrollen (9a, 9b, 14a, 14b) sich in der ersten Position befinden.

10. Verfahren nach einem der vorhergehenden Ansprüche,
**dadurch gekennzeichnet, dass**
die Formrollen (14a, 14b) des zweiten Formwerkzeugs (14) zu einer zweiten Position verlagerbar sind, in welcher diese durch einen zweiten Abstand (24) beabstandet sind, welcher kleiner ist als der erste Abstand (22), wobei in der zweiten Position durch die Formrollen (14a, 14b) eine Verformungskraft auf den Formabschnitt (13) des hohlzylindrischen Glasrohlings (11) beaufschlagbar ist, wodurch eine äußere Formgebung des Formabschnitts (13) realisierbar ist, wobei eine innere Formgebung des Formabschnitts (13) durch den Formstift (16) des dritten Formwerkzeugs (15) realisierbar ist.

11. Verfahren nach einem der vorhergehenden Ansprüche,
**dadurch gekennzeichnet, dass**
die Formrollen (9a, 9b) des ersten Formwerkzeugs (9) eine Breite (25) aufweisen, die kleiner oder gleich der Länge (L) des distalen Endabschnitts (4) sind und zu einer zweiten Position verlagerbar sind, in welcher diese durch einen zweiten Abstand (23) beabstandet sind, welcher kleiner ist als der erste Abstand (21), wobei in der zweiten Position durch die Formrollen (9a, 9b) eine Verformungskraft auf den distalen Endabschnitt (4) beaufschlagbar ist, wodurch eine Formgebung des distalen Endabschnitts (4) realisierbar ist und die innere Oberfläche des distalen Endabschnitts (4) an das Stechmittel (2) gedrückt wird.

12. Verfahren nach einem der vorhergehenden Ansprüche,
**dadurch gekennzeichnet, dass**
das erstmalige Formen des distalen Endabschnitts (4) in Schritt bb) und das weitere Formen des distalen Endabschnitts (4) in Schritt d) durch das erste Formwerkzeug (9) erfolgt.

13. Verfahren nach einem der vorhergehenden Ansprüche,
**dadurch gekennzeichnet, dass**
der Formstift (16) aus einem nichtmetallischen Material besteht, beispielswese einer technischen Keramik, einem keramikartigen Material oder glasartigem Kohlenstoff besteht.

14. Verfahren nach einem der vorhergehenden Ansprüche,
**dadurch gekennzeichnet, dass**
nach dem Schritt d) der mit dem Stechmittel (2) versehene Spritzenkörper (3) abgekühlt und / oder einem Sterilisierungsprozess zugeführt wird.

15. Spritze (1) mit einem Stechmittel (2) hergestellt durch ein Verfahren nach einem der vorhergehenden Ansprüche.

## Claims

1. A method for producing a syringe (1) having a piercing means (2), comprising the following steps:
a) providing a syringe body (3) having a distal end section (4), which comprises an inner channel (5) that discharges at a distal opening (6), wherein the distal end section (4) is in a formable state;
b) providing a piercing means (2);
c) inserting a proximal section (7) of the piercing means (2) through the distal opening (6) into the inner channel (5) of the distal end section (4);
d) shaping the distal end section (4) by means of a first shaping tool (9) in such a way that an inner surface (10) of the distal end section (4) contacts at least portions of the piercing means (2), as a result of which at least portions of the piercing means (2) are secured.

2. The method according to claim 1,
**characterised in that** producing the syringe body (3) comprises the following steps:
aa) providing a hollow, cylindrical glass preform (11), which extends along an axial direction (X) and has at least one open distal end (12), wherein the glass preform (11) features a shaped section (13) that extends from the open distal end (12) in a radial direction (X) and is in a formable state;
bb) shaping the shaped section (13) to the preferably cone-shaped distal end section (4) of the syringe body (3) by means of a second (14) and a third shaping tool (15).

3. The method according to claim 2,
**characterised in that**
step bb) comprises the following sub-steps:
(1) providing the second shaping tool (14), by means of which at least the shaped section (13) of the hollow-cylindrical glass preform (11) is able to be shaped;
(2) providing a third shaping tool (15), which features a shaping pin (16);
(3) inserting the shaping pin (16) through the open distal end (12) of the hollow-cylindrical glass preform (11) into the shaped section (13) thereof;
(4) shaping the shaped section (13) by means of the second shaping tool (14) such that the inner surface (10) of the shaped section (13) fits against the shaping pin (16), as a result of which the shaped section (13) forms the channel (5);
(5) removing the shaping pin (16) from the channel (5).

4. The method according to any of the preceding claims,
**characterised in that**
the shaping of the distal end section (4) in step d) takes place immediately after the syringe body (3) is produced, or the syringe body (3) is initially subjected to an additional process, in which the distal end section (4) is heated so that it is again transformed into a formable state.

5. The method according to any of the preceding claims,
**characterised in that**,
the piercing means (2) is rotatably mounted in a holding device (17), particularly in steps b) and c).

6. The method according to any of the preceding claims,
**characterised in that**
the distal end section (4) has a length (L), wherein the shaping in step d) takes place along a sub-section (18) of the length (L), as a result of which a contact section (19) of the channel or rather the inner surface (10) of the distal end section (4) contacts and at least rudimentarily secures the piercing means (2), thereby achieving complete contact around the circumference (U) of the piercing means (2) with the inner surface (10) of the distal end section (4).

7. The method according to claim 6,
**characterised in that**,
following the shaping in step d), a preferably organic adhesive is introduced through the distal opening (6) into a distal section (20) of the channel (5), thereby securing the piercing means (2) in the distal end section (4) such that a pullout force of at least 30 N is achieved without the use of an additional adhesive, wherein the distal section (20) of the channel (5) is limited by the distal opening (6) and the contact section (19) of the channel (5).

8. The method according to any of claims 1 to 5,
**characterised in that**
the shaping in step d) takes place across essentially the entire length (L) of the distal end section (4), as a result of which a contact section (19) of the channel (5) or rather the inner surface (10) of the distal end section (4) contacts and secures the piercing means (2) such that a pullout force of at least 30 N is achieved without the use of an additional adhesive, thereby achieving complete contact around the circumference (U) of the piercing means (2) with the inner surface (10) of the distal end section (4).

9. The method according to any of the preceding claims,
**characterised in that**
the first (9) and/or the second shaping tool (14) comprise two shaping rollers (9a, 9b, 14a, 14b) spaced apart from one another, wherein, in a first position, the shaping rollers (9a, 9b, 14a, 14b) are spaced apart by a first distance (21, 22), and wherein the distal end section (4) is displaceable between the shaping rollers (9a, 9b, 14a, 14b) when the shaping rollers (9a, 9b, 14a, 14b) are located in the first position.

10. The method according to any of the preceding claims,
**characterised in that**
the shaping rollers (14a, 14b) of the second shaping tool (14) are displaceable into a second position, in which they are spaced apart by a second distance (24), which is smaller than the first distance (22), wherein, in the second position, a deforming force is able to be applied to the shaped section (13) of the hollow-cylindrical glass preform (11) by means of the shaping rollers (14a, 14b), as a result of which the external shaping of the shaped section (13) can be accomplished, and wherein an internal shaping of the shaped section (13) can be accomplished by means of the shaping pin (16) of the third shaping tool (15).

11. The method according to any of the preceding claims,
**characterised in that**
the shaping rollers (9a, 9b) of the first shaping tool (9) have a width (25), which is smaller than or equal to the length (L) of the distal end section (4), and they are displaceable into a second position, in which they are spaced apart by a second distance (23), which is smaller than the first distance (21), wherein, in the second position, a deforming force is able to be applied to the distal end section (4) by means of the shaping rollers (9a, 9b), as a result of which a shaping of the distal end section (4) is able to be accomplished, and the inner surface of the distal end section (4) is pressed against the piercing means (2).

12. The method according to any of the preceding claims,
**characterised in that**
the initial shaping of the distal end section (4) in step bb) and the further shaping of the distal end section (4) in step d) takes place by means of the first shaping tool (9).

13. The method according to any of the preceding claims,
**characterised in that**
the shaping pin (16) consists of a non-metallic material, for example a technical ceramic, a ceramic-like material, or glass-like carbon.

14. The method according to any of the preceding claims,
**characterised in that**,
following step d), having been provided with the piercing means (2), the syringe body (3) is cooled and/or subjected to a sterilisation process.

15. A syringe (1) having a piercing means (2), which is produced by means of a method according to any of the preceding claims.

## Revendications

1. Procédé de fabrication d'une seringue (1) avec un moyen de perçage (2), le procédé comportant les étapes suivantes :
a) se procurer un corps de seringue (3) avec une section d'extrémité distale (4), laquelle présente un canal interne (5) qui débouche dans une ouverture distale (6), la section d'extrémité distale (4) se trouvant dans un état façonnable ;
b) se procurer un moyen de perçage (2) ;
c) introduire une section proximale (7) du moyen de perçage (2) à travers l'ouverture distale (6) dans le canal interne (5) de la section d'extrémité distale (4) ;
d) façonner la section d'extrémité distale (4) au moyen d'un premier outil de façonnage (9) de telle sorte qu'une surface interne (10) de la section d'extrémité distale (4) vienne en contact au moins par sections avec le moyen de perçage (2), ce par quoi le moyen de perçage (2) est fixé au moins par sections.

2. Procédé selon la revendication 1,
**caractérisé par le fait que**
la fabrication du corps de seringue (3) comporte les étapes suivantes :
aa) se procurer une préforme en verre (11) en forme de cylindre creux, s'étendant le long d'une direction axiale (X), avec au moins une extrémité distale ouverte (12), la préforme en verre (11) présentant une section de façonnage (13), qui s'étend en direction axiale (X) en partant de l'extrémité distale ouverte (12) et qui se trouve dans un état façonnable ;
bb) façonner la section de façonnage (13) en la section d'extrémité distale (4), de préférence en forme conique, du corps de seringue (3) au moyen d'un deuxième (14) et d'un troisième (15) outil de façonnage.

3. Procédé selon la revendication 2,
**caractérisé par le fait que**
l'étape bb) comporte les sous-étapes suivantes :
(1) se procurer le deuxième outil de façonnage (14), au moyen duquel au moins la section de façonnage (13) de la préforme en verre (11) en forme de cylindre creux est déformable ;
(2) se procurer un troisième outil de façonnage (15), lequel présente une broche de façonnage (16) ;
(3) introduire la broche de façonnage (16) à travers l'extrémité distale ouverte (12) de la préforme en verre (11) en forme de cylindre creux dans sa section de façonnage (13);
(4) façonner la section de façonnage (13) au moyen du deuxième outil de façonnage (14) de telle sorte que la surface interne (10) de la section de façonnage (13) s'applique contre la broche de façonnage (16), ce par quoi la section de façonnage (13) forme le canal (5) ;
(5) retirer la broche de façonnage (16) du canal (5).

4. Procédé selon l'une des revendications précédentes,
**caractérisé par le fait que**
le façonnage de la section d'extrémité distale (4) à l'étape d) a lieu directement après la fabrication du corps de seringue (3) ou le corps de seringue (3) est tout d'abord amené à un autre procédé dans lequel la section d'extrémité distale (4) est réchauffée, de telle sorte que celle-ci est convertie en un état façonnable.

5. Procédé selon l'une des revendications précédentes,
**caractérisé par le fait que** le moyen de perçage (2), en particulier dans les étapes b) et c), est monté apte à tourner dans un dispositif de maintien (17).

6. Procédé selon l'une des revendications précédentes,
**caractérisé par le fait que**
la section d'extrémité distale (4) présente une longueur (L), le façonnage à l'étape d) ayant lieu le long d'une sous-section (18) de la longueur (L), ce par quoi une section de contact (19) du canal respectivement de la surface interne (10) de la section d'extrémité distale (4) vient en contact avec et fixe le moyen de perçage (2) au moins de façon rudimentaire, le moyen de perçage (2) étant complètement le long de sa périphérie (U) en contact avec la surface interne (10) de la section d'extrémité distale (4).

7. Procédé selon la revendication 6,
**caractérisé par le fait qu'**
après le façonnage à l'étape d), un adhésif, de préférence organique, est introduit à travers l'ouverture distale (6) dans une section distale (20) du canal (5), ce par quoi le moyen de perçage (2) est fixé dans la section d'extrémité distale (4), de telle sorte qu'une force d'arrachage d'au moins 30 N est obtenue sans l'utilisation d'un autre adhésif, la section distale (20) du canal (5) étant délimitée par l'ouverture distale (6) et la section de contact (19) du canal (5).

8. Procédé selon l'une des revendications 1 à 5,
**caractérisé par le fait que**
le façonnage à l'étape d) a lieu sur sensiblement toute la longueur (L) de la section d'extrémité distale (4), ce par quoi une section de contact (19) du canal (5)) respectivement de la surface interne (10) de la section d'extrémité distale (4) vient en contact avec et fixe le moyen de perçage (2), de telle sorte qu'une force d'arrachage d'au moins 30 N est obtenue sans l'utilisation d'un autre adhésif, le moyen de perçage (2) étant complètement le long de sa périphérie (U) en contact avec la surface interne (10) de la section d'extrémité distale (4).

9. Procédé selon l'une des revendications précédentes,
**caractérisé par le fait que**
le premier (9) et/ou le deuxième (14) outil de façonnage comportent deux rouleaux de façonnage (9a, 9b, 14a, 14b) espacés l'un de l'autre, les rouleaux de façonnage (9a, 9b, 14a, 14b) dans une première position étant espacés d'une première distance (21, 22), la section d'extrémité distale (4) étant déplaçable entre les rouleaux de façonnage (9a, 9b, 14a, 14b) lorsque les rouleaux de façonnage (9a, 9b, 14a, 14b) se trouvent dans la première position.

10. Procédé selon l'une des revendications précédentes,
**caractérisé par le fait que**
les rouleaux de façonnage (14a, 14b) du deuxième outil de façonnage (14) sont déplaçables dans une seconde position dans laquelle ceux-ci sont espacés d'une seconde distance (24), laquelle est plus petite que la première distance (22), où, dans la seconde position, par les rouleaux de façonnage (14a, 14b), une force de déformation est applicable à la section de façonnage (13) de la préforme en verre (11) en forme de cylindre creux, ce par quoi un façonnage externe de la section de façonnage (13) est réalisable, un façonnage interne de la section de façonnage (13) étant réalisable par la broche de façonnage (16) du troisième outil de façonnage (15).

11. Procédé selon l'une des revendications précédentes,
**caractérisé par le fait que**
les rouleaux de façonnage (9a, 9b) du premier outil de façonnage (9) présentent une largeur (25) qui est inférieure ou égale à la longueur (L) de la section d'extrémité distale (4) et sont déplaçables dans une seconde position dans laquelle ceux-ci sont espacés d'une seconde distance (23), laquelle est plus petite que la première distance (21), où, dans la seconde position, par les rouleaux de façonnage (9a, 9b), une force de déformation est applicable à la section d'extrémité distale (4), ce par quoi un façonnage de la section d'extrémité distale (4) est réalisable et la surface interne de la section d'extrémité distale (4) est pressée contre le moyen de perçage (2).

12. Procédé selon l'une des revendications précédentes,
**caractérisé par le fait que**
le façonnage pour la première fois de la section d'extrémité distale (4) à l'étape bb) et le façonnage suivant de la section d'extrémité distale (4) à l'étape d) ont lieu au moyen du premier outil de façonnage (9).

13. Procédé selon l'une des revendications précédentes,
**caractérisé par le fait que**
la broche de façonnage (16) se compose d'un matériau non métallique, par exemple se compose d'une céramique technique, d'un matériau de type céramique ou de carbone vitreux.

14. Procédé selon l'une des revendications précédentes,
**caractérisé par le fait qu'**
après l'étape d), le corps de seringue (3) muni du moyen de perçage (2) est refroidi et/ou amené à un procédé de stérilisation.

15. Seringue (1) avec un moyen de perçage (2), fabriquée par un procédé selon l'une des revendications précédentes.
